# EUROPÄISCHE PATENTSCHRIFT

(11) **EP 2 237 750 B1**
(45) Veröffentlichungstag und Bekanntmachung des Hinweises auf die Patenterteilung: **26.10.2011**
(21) Anmeldenummer: 09703666.9
(22) Anmeldetag: 12.01.2009
(51) Int. Cl.: A61F 5/37

(54) **ARMABDUKTIONSORTHESE**
ARM ABDUCTION ORTHOTIC
ORTHÈSE D'ABDUCTION DU BRAS

(30) Priorität: 23.01.2008 DE 102008005729
(43) Veröffentlichungstag der Anmeldung: 13.10.2010
(60) Teilanmeldung: 11002902.2
(73) Patentinhaber: Otto Bock HealthCare GmbH, 37115 Duderstadt (DE)
(72) Erfinder: VOLLBRECHT, Matthias, 37412 Herzberg (DE); LIDOLT, Klaus, 37115 duderstadt (DE); KROLL-ORYWAHL, Olaf, 37085 Göttingen (DE)
(74) Vertreter: Stornebel, Kai
(86) Internationale Anmeldenummer: PCT/DE2009/000028
(87) Internationale Veröffentlichungsnummer: WO 2009/092348

(56) Entgegenhaltungen:
- DE-C- 582 933
- DE-U- 1 884 974
- DE-U1- 8 134 992
- US-A- 2 661 000
- US-A1- 2004 031 183

## Beschreibung

Die Erfindung betrifft eine Armabduktionsorthese mit einem zur Abstützung am Thorax des Orthesennutzers vorgesehenen Körperrahmen, an dem Befestigungsmittel zur Fixierung des Körperrahmens an dem Orthesennutzer angeordnet sind, und eine Auflageeinrichtung zur Auflage eines Armes des Orthesennutzers, die gelenkig an dem Körperrahmen befestigt ist und Mittel zur Festlegung des Armes an der Auflageeinrichtung aufweist.

Zur Versorgung von Patienten nach Verletzungen oder Operationen im Schulterbereich kann es notwendig sein, den Arm ruhig zu stellen. Die Ruhigstellung erfolgt dabei in Abhängigkeit von der Art der Verletzung bzw. Operation. Zur Ruhigstellung des Armes wurden früher Gipsverbände angelegt, die neben hygienischen Problemen und Komforteinschränkungen auch zu einer erhöhten Neigung der Versteifung des Schultergelenkes führen können. Darüber hinaus ist bei einem Gipsverband die einmal eingestellte Stellung unveränderbar, so dass bei einem Genesungsfortschritt keine einfache Veränderung erfolgen kann, sondern der Arme in einer neuen Stellung eingegipst werden muss.

Zur Versorgung von Patienten werden daher Abduktionsorthesen eingesetzt, die unterschiedlich ausgebildet sein können. Als einfachste Form ist ein so genanntes Abduktionskissen zu nennen, das an dem Torso des Patienten befestigt wird. Auf das Kissen wird der abzustützende Arm aufgelegt und daran fixiert. Solche Abduktionskissen sind zwar leicht anzulegen, bewirken jedoch nur eine geringe Stabilisierung und sind nur im begrenzten Maße an die individuellen Bedürfnisse und einen Genesungsfortschritt anpassbar.

Aus der WO 03/071994 A2 ist eine variable Armabduktionsorthese bekannt, das ähnlich einem Armabduktionskissen aufgebaut ist und aus einem Distal- und einem Proximalelement besteht. Ein Achselkeil ist höhenverstellbar an dem Distalelement oder Proximalelement angeordnet. Das Proximalelement und das Distalelement sind in einem Abstand zueinander verstellbar angeordnet, wobei der eingestellte Winkel über eine Raststelleneinrichtung fixiert werden kann. Aufgrund der kissenartigen Ausgestaltung sowohl des Proximalelementes als auch des Distalelementes ist diese Armabduktionsorthese sperrig.

Die DE 43 23 261 A1 beschreibt eine Orthese zur Fixation und/oder induzierten Bewegung einer Gliedmaße oder eines Teiles davon mit einem am Körper des Patienten zu befestigenden Gestell, das aus einer Thoraxspange und einer Beckenstütze besteht. Eine Strebe führt von der Thoraxspange zur Beckenstütze und weist an ihrem oberen Ende ein Scharnier auf, an dem eine Armschiene, die aus zwei Abschnitten besteht, schwenkbar gelagert ist. Die Schwenkachse des Scharniers verläuft rechtwinklig zur Längserstreckung der Strebe und angenähert parallel zur Thoraxspange. Zwischen der Strebe und der Armschiene ist eine Stützeinrichtung vorgesehen, die motorisch verstellbar ausgebildet ist. Je nach Drehrichtung des Motors wird der Abduktionswinkel vergrößert oder verkleinert. Auf diese Art und Weise kann das Schultergelenk automatisch bewegt werden, ohne Muskeln betätigen zu müssen, so dass das Schultergelenk nicht versteift. Eine solche Orthese ist relativ schwer und kostenaufwändig zu fertigen.

Die US 2004/0031183 A1 beschreibt eine Unterstützungseinrichtung des Armes beim Zielen während des Schießens. Ein Körperrahmen wird vor den Torso geschnallt. An dem Körperrahmen ist eine Armauflage zur Auflage des Oberarmes vorgesehen. Über einen Scharniermechanismus kann die Armauflage aus einer im Wesentlichen waagerechten, verriegelten in eine abgesenkte Stellung verlagert werden.

Die DE 582 933 C1 betrifft eine am Körper zu befestigende Armauflagerungsschiene mit Schultergelenk, bei der der Träger des Schultergelenks längs der oberen, um die Brust geführten Spange verschiebbar und verstellbar ist. Die Armauflagen werden durch Metallschalen ausgebildet.

Die US 2,661,000 B1 betrifft eine Auflageeinrichtung für einen Arm mit einer Körperschiene, die über Gurte an dem Torso befestigt ist. Ein Schultergelenk mit einem Träger für den Oberarm und einem winklig dazu schwenkbar gelagerten Unterarmträger ist gelenkig an der Körperschiene angeordnet. Eine Verstärkung stützt die Oberarmschiene gegenüber der Körperschiene ab.

Die DE 81 34 992 U1 beschreibe eine Armschiene zur Abstützung eines beispielsweise im Schultergelenk verletzten Armes in gehobener, vom Körper abgewinkelter Stellung, die im Wesentlichen aus einer Armauflage sowie einer gepolsterten Beckenstütze und einem , die beiden Teile verbindenden, ineinanderschiebbaren, zweitteiligen Führungsrohr und einem den Brustkorb umspannenden Haltegurt besteht. Die Armauflage ist als ein Rohrrahmen ausgebildet, der relativ zu dem Führungsrahmen verschoben und um die Längsachse des Führungsrohres herum verschwenkt werden kann.

Aufgabe der vorliegenden Erfindung ist es, eine leichte Armabduktionsorthese bereitzustellen, die komfortabel zu tragen, stabil und preiswert herzustellen ist.

Erfindungsgemäß wird diese Aufgabe durch eine Armabduktionsorthese mit den Merkmalen des Anspruchs 1 gelöst. Vorteilhafte Ausgestaltungen und Weiterbildungen der Erfindung sind in den Unteransprüchen beschrieben.

Die erfindungsgemäße Armabduktionsorthese mit einem zur Abstützung am Thorax des Orthesennutzer vorgesehenen Körperrahmen, an dem Befestigungsmittel zur Fixierung des Körperrahmens an dem Orthesennutzer angeordnet sind, und einer Auflageeinrichtung zur Auflage eines Armes des Orthesennutzers, die gelenkig an dem Körperrahmen befestigt ist und Mittel zur Festlegung des Armes an der Auflageeinrichtung aufweist, sieht vor, dass die Auflageeinrichtung einen Abstützrahmen mit zwei zueinander beabstandeten Rahmenprofilen aufweist, zwischen denen ein Auflagematerial angeordnet ist, und dass sich der Abstützrahmen an dem Körperrahmen über zumindest ein Stützelement abstützt. Durch die Ausbildung der Auflageeinrichtung als ein Abstützrahmen mit zwei zueinander beabstandeten Rahmenprofilen ist es möglich, eine hohe Stabilität der Auflageeinrichtung bei einer gleichzeitigen Leichtbauweise zu erreichen. Statt einer aufwändigen Verriegelungseinrichtung im Gelenk zwischen dem Abstützrahmen und dem Körperrahmen ist vorgesehen, dass ein Stützelement direkt zwischen dem Abstützrahmen und dem Körperrahmen angeordnet ist. Auf diese Art und Weise wird es möglich, die innerhalb der Auflageeinrichtung auftretenden Kräfte direkt und unmittelbar über den Abstützrahmen auf den Körperrahmen zu übertragen, so dass nur die Rahmenkonstruktion den Arm abstützt und trägt.

Die Erfindung sieht vor, dass das Auflagematerial flexibel ausgebildet und zwischen den Rahmenprofilen gespannt ist. Auf dem stabilen Rahmen kann das flexible Auflagematerial gespannt sein, auf dem der Arm des Orthesennutzers während der Nutzung der Armabduktionsorthese aufliegt. Der Arm des Orthesennutzers liegt somit nicht auf einer festen Abstützschale oder auf einer festen Schiene auf, sondern vielmehr auf einem flexiblen Material, so dass eine Druckstellenproblematik, insbesondere im Bereich des Ellenbogengelenkes vermieden werden kann, da der Arm nicht auf einer harten Schiene oder Abstützplatte aufliegen muss. Die Rahmenprortie des Körperrahmens und des Abstützrahmens können einen offenen oder geschlossenen Querschnitt aufweisen.

Bevorzugt ist vorgesehen, dass der Abstützrahmen geschlossen ausgebildet ist, um eine erhöhte Stabilität aufzuweisen. Ein geschlossener Rahmen wird durch ein umlaufendes Rahmenprofil oder durch einzelne Rahmenprofile gebildet, die miteinander verbunden sind, so dass sich ein geschlossener Streckenzug bildet. Ein geschlossener Streckenzug kann auch durch einzelne Rahmenteile gebildet werden, die miteinander verbunden sind, beispielsweise über Steckverbindungen. Rahmenteile können auch in ein Gelenk oder Gelenkteil eingesteckt oder daran befestigt werden, beispielsweise geklemmt oder formschlüssig festgelegt werden, um zusammen mit dem Gelenk oder Gelenkteil einen umlaufenden Streckenzug und damit einen geschlossenen Rahmen auszubilden. Das vordere, der Hand zugewandte Ende des Abstützrahmens ist bevorzugt in Bogenform ausgebildet, damit die Hand sich darum schließen kann oder damit ein Auflagekissen für die Handfläche auf dem Auflagematerial befestigt werden kann.

Der Abstützrahmen kann eine winkelige, z.B. rechtwinklige Kontur aufweisen, also im Wesentlichen L-förmig ausgebildet sein, um einen entsprechend abgewinkelten Arm zu umgeben bzw. um die Oberarm- und Unterarmknochen seitlich einzuschließen. Dadurch wird gewährleistet, dass kein Knochen, insbesondere der Ellenbogen, auf einem Rahmenteil aufliegt, sondern von dem Auflagematerial aufgenommen wird.

Der Körperrahmen kann ebenfalls eine geschlossene, im Wesentlichen ebene Kontur aufweisen, um eine sichere Abstützung und ausreichende Stabilität der Armabduktionsorthese durch eine kompakte Anlage an dem Thorax zu gewährleisten. Die ebene Kontur unterstützt die universelle Anwendbarkeit, indem aufgrund der flächigen Ausgestaltung des Körperrahmens dieser sowohl auf der rechten als auch auf der linken Thoraxseite angeordnet werden kann. Die Rahmenprofile oder das Rahmenprofil bilden auch hier einen geschlossenen Streckenzug, z.B. ein Rechteck oder eine Ellipse, die Ausführungen zu dem Abstützrahmen gelten entsprechend.

Um den Armabduktionswinkel einstellen zu können, ist vorgesehen, dass der Abstützrahmen und der Körperrahmen über ein Doppelgelenk, ein Filmscharnier oder zumindest ein Einhakgelenk gelenkig aneinander gelagert sind. Durch das Doppelgelenk, Filmscharnier oder ein entsprechendes Einhakelement ist es möglich, dass der Abstützrahmen sich in einem beliebigen Winkel zu dem Körperrahmen verschwenken lässt und im Extremfall eine Verschwenkbarkeit in einem Winkel von 360° ermöglicht. Dadurch ist es möglich, dass der Abstützrahmen parallel zu dem Körperrahmen geführt werden kann, was einem Abduktionswinkel von 0° entspricht. Diese Parallelität kann auf beiden Seiten des Körperrahmens verwirklicht werden, wodurch die beidseitige Anwendbarkeit der Armabduktionsorthese gewährleistet bleibt. Das Doppelgelenk kann auch aus zwei Filmscharnieren gebildet werden.

Eine Weiterbildung der Erfindung sieht vor, dass der Abstützrahmen mehrteilig ausgebildet und über Steckverbindungen montiert ist. Die erleichtert die Montage und eine Anpassung an unterschiedliche physiologische Gegebenheiten. Darüber hinaus besteht die Möglichkeit, dass bei einer mehrteiligen Ausbildung des Abstützrahmens eine Längenanpassung erfolgen kann, beispielsweise indem die Steckverbindungen in verschiedenen Abständen Rast- und Verriegelungseinrichtungen aufweisen, wodurch eine individuelle Anpassung der Armabduktionsorthese an den Patienten ermöglicht und erleichtert wird. Alternativ kann leicht über eine modulare Ausgestaltung des Abstützrahmens eine individuelle Fertigung erfolgen.

Eine Weiterbildung der Erfindung sieht vor, dass ein Unterarmteil des Abstützrahmens in einer Ebene liegt, die in einem Winkel zu der durch einen Oberarmteil gebildeten Ebene orientiert ist. Auf diese Art und Weise wird eine Außenrotation des Arms bewirkt, die für verschiedene Versorgungsfälle ratsam erscheint. Insbesondere bei einer mehrteiligen Ausgestaltung des Abstützrahmens ist es möglich, dass in der Umgebung des Ellenbogenbereiches Winkelstücke eingefügt werden, die eine Verkippung des Unterarmteils relativ zu der Ebene der Oberarmauflage bewirkt, so dass durch Auswechseln der Adapterstücke eine angepasste Außenrotation eingestellt werden kann. Alternativ dazu ist vorgesehen, dass der Abstützrahmen einteilig im Bereich der Abstützung des Oberarms und des Unterarms ausgebildet ist und die Außenrotation fixiert eingestellt bleibt. Eine dritte Möglichkeit sieht vor, dass ein auf dem Unterarmteil des Abstützrahmens befestigtes Auflageelement die Außenrotation bewirkt, wobei das Auflageelement beispielsweise in Keilform ausgebildet sein kann, um die Außenrotation zu bewirken. Ebenfalls ist es möglich, dass ein zweites Polsterteil auf dem Unterarmteil aufgebracht ist, das durch eine verschiebliche Anordnung von Winkeln oder eine unterschiedliche Ausgestaltung der Winkel in seiner Stellung zu dem Oberarmteil veränderlich festgelegt wird.

Der Körperrahmen kann ebenfalls mit einem flexiblen Bezug bespannt sein, bevorzugt mit einer Polsterung, wobei der Polsterbezug bevorzugt aus einem kaschierten Schaumstoff besteht, der einerseits ein gewisses Maß an Festigkeit und andererseits ein hohes Maß an Polsterung bietet. Über die Kaschierung des Schaumstoffes ist es möglich, beliebige Anlageflächen für Hakenelemente eines Klettverschlusses bereitzustellen. Das Auflagematerial des Abstützrahmens kann ebenfalls aus einem gepolsterten Bezugstoff bestehen und ist insbesondere bevorzugt aus einem kaschierten Schaumstoff ausgeführt. Der Schaumstoff für beide Rahmen ist bevorzugt aufgrund einer offenporigen Schaumstruktur atmungsaktiv und kann abnehmbar an dem Rahmen befestigt sein. Dies erleichtert eine Waschbarkeit und fördert eine hygienische Handhabung der Armabduktionsorthese.

Darüber hinaus kann durch einen abnehmbaren Bezug eine Weiterverwendbarkeit der Rahmenkonstruktion erfolgen, da lediglich das Abstützmaterial und der Bezug des Abstütz- und Körperrahmens entfernt werden müssen.

An dem Körperrahmen kann ein Stabilisierungsabschnitt angeordnet sein, der ebenfalls gepolstert und mit einem kaschierten Schaumstoff an der Außenseite versehen ist. Stabilisierungselemente können in dem Stabilisierungsabschnitt angeordnet sein, beispielsweise Kunststoffstäbe oder andere Profile, um eine Anlage an den Thorax zu ermöglichen. Grundsätzlich ist eine Anordnung von Stabilisierungsabschnitten beidseitig an dem Körperrahmen möglich, bevorzugt eine symmetrische Anordnung, um die Rechts-Links-Anwendbarkeit beizubehalten.

Das Stützelement zwischen dem Körperrahmen und dem Abstützrahmen kann als Keil, insbesondere als ein formstabiler Spritzguss- oder Schaumstoffkeil aus Kunststoff mit darauf aufgebrachten Hakenelementen ausgebildet sein. Der Keil stützt sich dann über den Rahmen bzw. die Rahmenprofile an dem Abstützrahmen und dem Körperrahmen ab. Bevorzugt ist der Keil dreieckig ausgebildet, wobei die Ausgestaltung des Winkelbereichs so vorgesehen ist, dass eine Vielzahl der üblichen Abduktionswinkeleinstellungen damit realisiert werden können, insbesondere eine Winkeleinstellbarkeit von 15°, 30°, 45°, 60°, 75° und 90°. In der 0° Stellung wird kein Keil verwendet, sondern bevorzugt ein beidseitiges Klettelement, um den Abstützrahmen an dem Körperrahmen zu fixieren.

Alternativ ist vorgesehen, dass zumindest eine an dem Rahmen bzw. an den Rahmen- oder Rohrprofilen festlegbare Strebe vorgesehen ist, über die sich die Rahmen aneinander abstützen. Die Strebe kann an unterschiedlichen Punkten des oder der Rahmen angeordnet werden, um den gewünschten Abduktionswinkel einstellen zu können. Die Strebe oder die Streben können in Ausnehmungen innerhalb des Rahmenprofils bzw. der Rahmenprofile eingerastet werden. Ebenfalls können andere Befestigungsmöglichkeiten an dem Bezug oder dem Abstützmaterial vorgesehen sein, um eine sichere Abstützung des Abstützrahmens an dem Körperrahmen durch eine direkte Kopplung des Kraftflusses über eine Strebe zu ermöglichen. Durch die Strebe bzw. das separate Stützelement kann das Gelenk zwischen dem Abstützrahmen und dem Körperrahmen sehr klein ausgeführt werden, so dass bei einer Abduktion von 0° keine sperrigen Rasteinrichtungen im Gelenkbereich entfernt werden müssen. Darüber hinaus baut das Gelenk mit dem Einsatz eines Abstützelementes leichter und kleiner. Ebenfalls ist es möglich, dass das Stützelement als ein Flachelement ausgebildet ist, das den Abstützrahmen an dem Körperrahmen abstützt.

An dem Abstütz- und Körperrahmen können Verstärkungen angeordnet oder ausgebildet sein, um die notwendige Torsionssteifigkeit zu erhöhen. Beispielsweise können schwenkbare Querverstrebungen zwischen zwei parallelen Rahmenabschnitten angeordnet sein, so dass eine Verlagerung der Rahmenabschnitte aufeinander zu oder voneinander weg verhindert oder erschwert wird, ohne dass eine unmittelbare Auflage des Armes auf den Verstrebungen erfolgt.

Die Rahmenprofile können aus Metall oder Kunststoff ausgebildet sein und neben einem runden auch einen ovalen oder andere Querschnitte aufweisen, die in Abhängigkeit von den mechanischen Belastungen gewählt werden.

Nachfolgend werden Ausführungsbeispiele der Erfindung anhand der beigefügten Figuren näher erläutert. Es zeigen:
- Figur 1 -: eine angelegte Armabduktionsorthese in einer ersten Stellung;
- Figur 2 -: eine Armabduktionsorthese gemäß Figur 1 in einer zweiten Stellung;
- Figur 3 -: eine Armabduktionsorthese in abgelegtem Zustand mit teilweise entfernten Polstern;
- Figur 4 -: eine Rahmenkonstruktion in einem ausgebreiteten Zustand;
- Figur 5 -: eine Rahmenkonstruktion gemäß Figur 4 in angewinkeltem Zustand;
- Figur 6 -: eine Rahmenkonstruktion gemäß Figur 4 mit einem Außenrotationsmodul; sowie
- Figur 7 -: eine angewinkelte Armkonstruktion gemäß Figur 6.

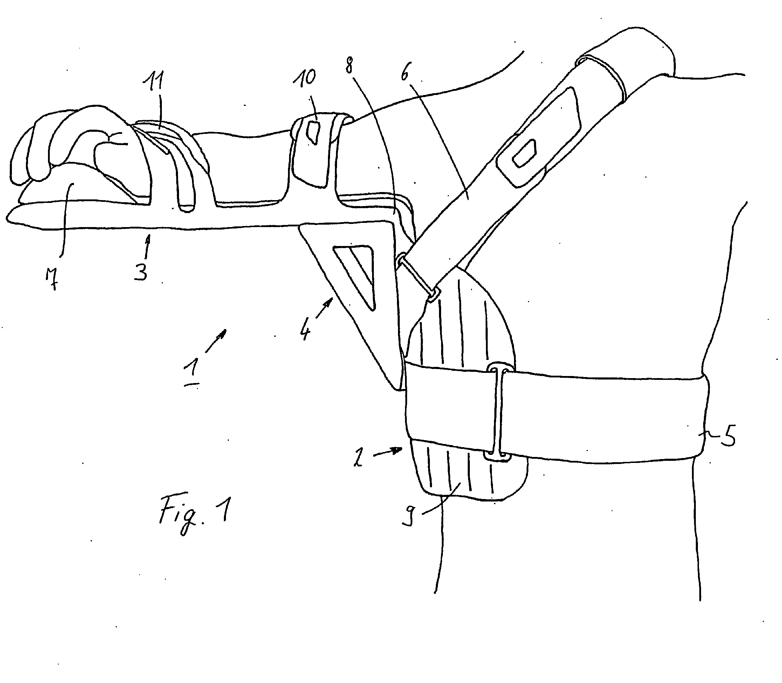

Die Figur 1 zeigt eine Armabduktionsorthese 1 in einer ersten Stellung. Die Armabduktionsorthese 1 weist eine körperseitige Abstützeinrichtung 2 auf, die über Befestigungsmittel 5, 6 an dem Thorax eines Orthesennutzers festlegbar ist. Die Befestigungsmittel 5, 6 bestehen aus Gurten, die einmal um den Thorax und über die Schulter des Orthesennutzers geführt sind. Bevorzugt sind diese Befestigungsmittel 5, 6 als in der Länge verstellbare Riemen oder Gurte ausgebildet, die gepolstert sind, um ein angenehmes Tragen zu ermöglichen. Ebenfalls können diese Befestigungsmittel 5, 6 mit einer gewissen Elastizität ausgebildet sein, um das Atmen zu erleichtern und geringe Relativbewegungen zuzulassen.

Weiterhin weist die Armabduktionsorthese 1 eine Abstützeinrichtung 3 für den Arm des Orthesennutzers auf. Die Abstützeinrichtung 3 ist über ein Gelenk 8 an der thoraxseitigen Abstützeinrichtung 2 verschwenkbar gelagert. In der Figur 1 ist die Abstützeinrichtung so ausgerichtet, dass der Arm des Orthesennutzers in einem im Wesentlichen rechten Winkel zu der Körperlängsachse orientiert ist, so dass die Auflageeinrichtung 3 im Wesentlichen horizontal ausgerichtet ist, wenn der Orthesennutzer steht oder aufrecht sitzt. Zwischen der Auflageeinrichtung 3 und der thoraxseitigen Abstützeinrichtung 2 ist ein Stützelement 4 angeordnet, das in dem vorliegenden Ausführungsbeispiel als ein Keil ausgebildet ist. Das Stützelement 4 kann auf verschiedene Arten und Weisen an der Armabduktionsorthese 1 befestigt sein. In dem dargestellten Ausführungsbeispiel sind sowohl die Auflageeinrichtung 3 als auch die Abstützeinrichtung 2 mit einem gepolsterten, kaschierten Überzug versehen, der weiter unten näher beschrieben wird. Dieser Überzug eignet sich dazu, um mit Hakenelementen in Eingriff zu treten, um einen Klettverschluss bzw. eine Klettverbindung zwischen dem Stützelement 4 und der Auflageeinrichtung 3 sowie der Abstützeinrichtung 2 zu verwirklichen. Dabei sind die Hakenelemente an vielen Stellen an dem oder in dem Stützelement 4 angeordnet, so dass sich eine beliebige Positionierung an der gesamten Oberfläche der Armabduktionsorthese 1 realisieren lässt.

Weiterhin sind an der Auflageeinrichtung 3 Befestigungsmittel 10, 11 zur Fixierung des Armes auf der Auflageeinrichtung 3 vorgesehen. Diese Befestigungsmittel sind als verstellbare Riemen, bevorzugt mit Klettversschluss, ausgebildet und sind beidseitig einsetzbar. An dem distalen Ende der Auflageeinrichtung 3 ist ein Handpolster 7 in gerundeter Form angeordnet. Auch dieses Polster 7 kann über eine Klettverbindung an der Abstützeinrichtung 3 befestigt werden, wodurch eine individuelle Anpassung an den Orthesennutzer möglich ist. Ebenfalls kann die Auflage 7 leicht ausgewechselt und gereinigt werden.

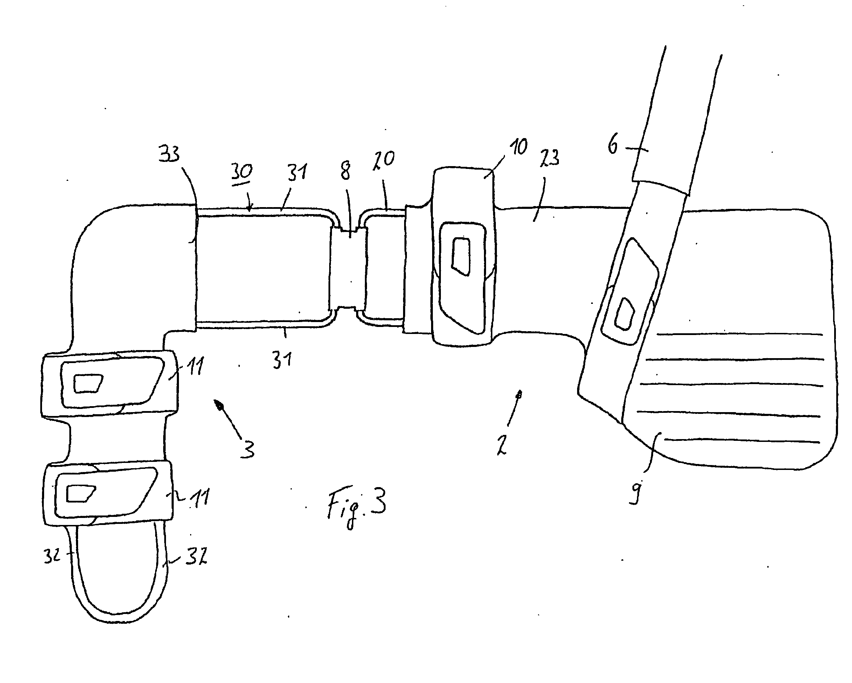

An der thoraxseitigen Abstützeinrichtung 2 ist zumindest ein Stabilisierungsabschnitt 9 vorgesehen, der sich zumindest teilweise frontal oder dorsal um den Thorax herumlegt, um eine weitergehende Stabilisierung zu ermöglichen. In dem Stabilisierungsabschnitt 9 können Kunststoffstäbe oder andere Stabilisierungselemente angeordnet sein, beispielsweise eingesteckt oder eingeschweißt, so dass eine Formstabilität des Stabilisierungsabschnittes 9 gegeben ist. Der Stabilisierungsabschnitt 9 kann ebenfalls gepolstert ausgebildet sein und ist bevorzugt mit einem kaschierten Schaumstoff überzogen, so dass auch an dem Stabilisierungsabschnitt 9 an beliebiger Position das Befestigungsmittel 5 festgelegt werden kann. Innerhalb der thoraxseitigen Abstützeinrichtung 2 ist ein Rahmen angeordnet, der nicht zu erkennen ist, da er innerhalb der Polsterung angeordnet ist. Der Aufbau wird unter Figur 3 näher beschrieben werden.

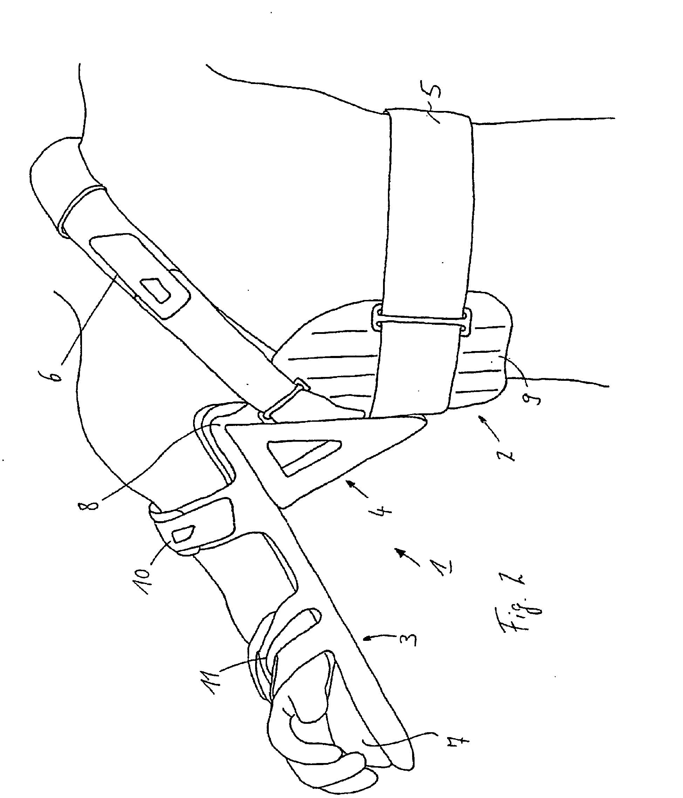

In Figur 2 ist die Armabduktionsorthese 1 gemäß Figur 1 mit einem geringeren Abduktionswinkel dargestellt. Dies wird dadurch erreicht, dass das Stützelement 4, das als rechtwinkliges Dreieck ausgebildet ist, mit der Hypotenuse in Richtung auf den Thorax gedreht an der Armabduktionsorthese 1 befestigt ist. Durch eine weitere Drehung des Stützelementes 4 kann ein noch geringerer Abduktionswinkel erreicht werden. Bevorzugt sind Abduktionswinkel von 15°, 30°, 45°, 60°, 75° und 90° vorzusehen, so dass eine Anpassung des Abduktionswinkels an die Erfordernisse für den jeweiligen Orthesennutzer erfolgen kann. Sofern das Stützelement 4 vollständig weggelassen wird, kann ein Abduktionswinkel von 0° realisiert werden, der Arm liegt dann am Thorax fixiert an.

Das Stützelement 4 stützt sich an Rahmen ab, die innerhalb der Auflageeinrichtung 3 und der Abstützeinrichtung 2 angeordnet sind. Alternativ zu der Ausbildung des Stützelementes 4 als ein dreieckiger Keil können andere Formen des Stützelementes 4 vorgesehen sein. Ebenfalls ist es vorgesehen, dass das Stützelement 4 aus einer oder mehreren Streben bestehen kann, die jeweils an den Rahmen befestigt werden. Auch ist es möglich, dass die Einzelstreben sich überkreuzend angeordnet sind und an dem Bezugsmaterial festgelegt sind, beispielsweise über eine Klettverbindung. Der Keil 4 kann aus Kunststoff hergestellt sein, z.B. aus einem Schaumstoff, der ausreichend formstabil ist. Ebenfalls kann der Keil 4 als Spritzgussteil ausgebildet sein.,

In der Figur 3 ist in Draufsicht eine nicht angelegte, flach ausgebreitete Armabduktionsorthese gemäß der Figuren 1 und 2 dargestellt. Die thoraxseitige Abstützeinrichtung 2 weist dabei einen eingearbeiteten Körperrahmen 20 auf, dessen Aufbau detaillierter anhand der nachfolgenden Figuren näher erläutert werden wird. Der Körperrahmen 20 ist mit einem Bezug 23 umhüllt, der aus einem gepolsterten Schaumstoff mit einer aufkaschierten Flauschschicht ausgebildet ist. Der Bezug 23 ist zurückgeschoben, um die Rahmenkonstruktion zu zeigen, im angelegten Zustand, wie er in den Figuren 1 und 2 gezeigt ist, erstreckt sich der Bezug 23 über das Gelenk 8. An dem Bezug 23 sind die Befestigungseinrichtungen befestigt oder daran ausgebildet, nämlich ein Oberarmbefestigungsgurt 10 und ein Schultergurt 6, ein Beckengurt 5 ist nicht dargestellt. Ebenfalls ist ein Stabilisierungsabschnitt 9 an dem Körperrahmen 20 angeordnet, vorliegend nur einseitig, so dass dieser Stabilisierungsabschnitt 9 im angelegten Zustand frontal an dem Thorax des Orthesennutzers anliegt. In dem Stabilisierungsabschnitt 9 können Kunststoffstäbe oder andere Verstärkungseinrichtungen eingearbeitet sein. Der Stabilisierungsabschnitt 9 ist im vorliegenden Fall einstückig mit dem Bezug 23 ausgebildet, alternativ kann der Stabilisierungsabschnitt 9 auch auf andere Art und Weise an dem Körperrahmen 20 oder an dem Bezug 23 befestigt sein. Ebenfalls kann eine dorsale Orientierung eines Stabilisierungsabschnittes 9 alternativ oder zusätzlich vorgesehen werden.

In der Figur 3 ist ebenfalls die Auflageeinrichtung 3 für den Arm des Orthesennutzers zu erkennen. Die Auflageeinrichtung 3 weist einen Abstützrahmen 30 auf, der aus zueinander beabstandeten Rahmenprofilen ausgebildet ist, zwischen denen ein flexibles Auflagematerial 33 gespannt ist. Dieses Auflagematerial 33 kann ebenfalls aus einem gepolsterten Schaumstoff mit einer Flauschoberfläche bestehen und über den Abstützrahmen 30 übergezogen sein. An dem Auflagematerial 33 sind Befestigungseinrichtungen 11 für den Unterarm befestigt oder ausgebildet, die Befestigungseinrichtungen 10 zum Fixieren des Oberarmes sind in dieser Darstellung zurückgeschoben, um den inneren Aufbau der Auflageeinrichtung 3 darstellen zu können.

Das flexible Auflagematerial 33 kann entweder elastisch oder unelastisch ausgebildet sein, zur Erhöhung des Komforts ist eine leichte Elastizität vorteilhaft. Da auf dem Auflagematerial 33 der Arm des Orthesennutzers aufliegt, gegebenenfalls über einen Zeitraum von bis zu acht Wochen, ist das Auflagematerial 33 bevorzugt aus einem atmungsaktiven und pflegeleichten Material hergestellt, das abnehmbar an dem Abstützrahmen 30 festgelegt ist, um es waschen zu können. Statt eines Überzuges, wie in der Figur 3 dargestellt, kann das Auflagematerial 33 auch einlagig zwischen den Rahmenprofilen angeordnet werden, beispielsweise indem die Rahmenprofile eingenäht oder in Schlaufen eingesteckt werden.

Zwischen dem Abstützrahmen 30 und dem Körperrahmen 20 ist ein Gelenk 8 in Gestalt eines Doppelgelenkes ausgebildet. Ein Gelenkteil ist schwenkbar sowohl an dem Abstützrahmen 30 als auch an dem Körperrahmen 20 befestigt, so dass der Abstützrahmen 30 beidseitig flach auf die Abstützeinrichtung 2 gelegt werden kann. Damit ist es möglich, einen Abduktionswinkel von 0° zu realisieren, unabhängig davon, ob die Armabduktionsorthese 1 rechts oder links getragen wird. Die Befestigungsmittel 5, 6, 10, 11 sind so ausgebildet, dass sie beidseitig wirksam sind, also dass auch ein Umschlagen in die Zeichnungsebene gemäß Figur 3 möglich ist. Somit ist es vorgesehen, dass die Armabduktionsorthese 1 einfach sowohl für den rechten Arm als auch für den linken Arm eingesetzt werden kann.

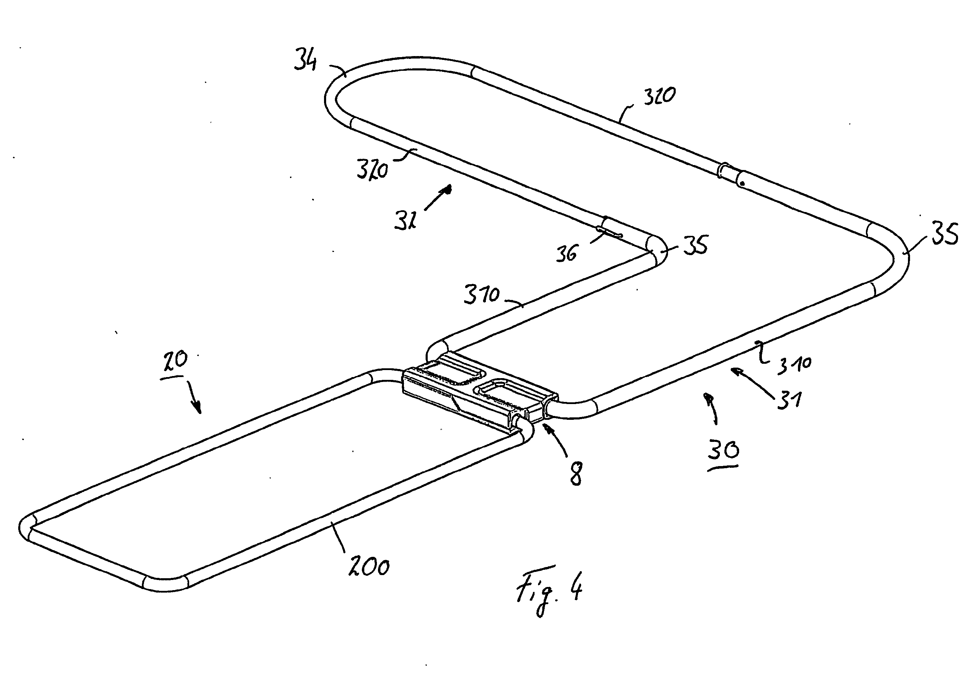

In der Figur 4 ist die Rahmenkonstruktion der Armabduktionsorthese 1 ohne Bezüge dargestellt. Der Körperrahmen 20 ist als ein geschlossener Rahmen aus Rahmenprofilen 200 oder einem Rahmenprofil 200 ausgebildet und schwenkbar an dem Gelenk 8 gelagert. In der dargestellten Ausführungsform ist der Körperrahmen 20 aus einem runden Metallprofil durch Biegen gefertigt, alternative Querschnittsformen des Rahmenprofils 200 sind möglich, z.B. eckige geschlossene Profilquerschnitte oder offene Profilquerschnitte. Ebenfalls ist es vorgesehen, dass der Körperrahmen 20 aus einem Spritzgussteil oder mehreren Spritzgussteilen hergestellt ist, gegebenenfalls auch aus einem Kunststoff, wobei die Dimensionierung des Rahmenprofils 200 bzw. der Rahmenprofile 200 in Abhängigkeit von den Materialeigenschaften zu wählen ist. Ebenfalls ist es möglich und vorgesehen, dass Verstärkungen innerhalb des Körperrahmens 20 angeordnet sind, um Torsionen oder Biegungen zu vermeiden. Der Körperrahmen 20 weist eine im Wesentlichen rechteckige Kontur auf, abweichende Konturen sind möglich, wobei bei einer Klappsymmetrie zur Längserstreckung die Eignung zum Rechts-Links-Tragen beibehalten wird.

Der Abstützrahmen 30 ist im dargestellten Ausführungsbeispiel zweiteilig aufgebaut und weist einen Oberarmteil 31 mit zwei im Wesentlichen zueinander parallel ausgerichteten Rahmenprofilen 310 auf, die Bögen 35 aufweisen, so dass sich eine L-förmige Kontur ergibt. An den distalen Enden der Rahmenprofile 310 ist ein Unterarmteil 32 mit entsprechenden Rahmenprofilen 320 befestigt. Die Befestigung erfolgt über eine Steckverbindung, die über einen Federstift 36 gesichert ist. Das distale Ende 34 des Unterarmteils 32 ist gerundet ausgeführt, so dass sich eine im Wesentlichen U-förmige Kontur ausbildet. Alternative Konturen sind möglich, ebenfalls ist es vorgesehen, dass zwischen den Rahmenprofilen 310, 320 Verstrebungen vorhanden sind, um eine Torsionssteifigkeit zu gewährleisten und Verbiegungen vorzubeugen. Die Rahmenprofile 310, 320 sind zueinander beabstandet, so dass sich ein im Wesentlichen L-förmiger Freiraum zwischen den Rahmenprofilen 310, 320 ausbildet. Insbesondere im Bereich der Bogenstücke 35 bildet sich ein relativ großer Freiraum aus, der zur Auflage des Ellenbogens auf dem dazwischen gespannten oder angeordneten Auflagematerial vorgesehen ist. Durch das Aufliegen des Ellenbogens auf dem flexiblen Auflagematerial 33 entfällt eine Druckstellenproblematik im Bereich des Ellenbogengelenkes. Durch die modulartige Konstruktion mit einem einsteckbaren Unterarmteil 32 kann eine einfache Längenanpassung an unterschiedliche Patienten erfolgen. Auch ist es möglich, die Bogenstücke 35 als separate Module auszubilden, so dass der Oberarmteil 31 auch in der Länge variabel ausgestaltet werden kann.

Auch der Abstützrahmen 30 ist schwenkbar an dem Gelenk 8 gelagert, so dass sich ein Doppelgelenk ausbildet, wodurch eine problemlose Verschwenkung über einen sehr großen Winkelbereich möglich ist. Die Konstruktion des Abstützrahmens 30 aus einem Rohrmaterial bietet bei einer sehr leichten Konstruktion ein hohes Maß an Stabilität. Die Rahmenprofile 20, 310, 320 können sowohl aus Hohlmaterial als auch aus Vollmaterial ausgebildet sein und belastungsangepasste Querschnitte aufweisen. In der Figur 4 ist eine Ausführungsform dargestellt, in der der Körperrahmen 20 und der Abstützrahmen 30 in der ausgeklappten Stellung in einer Ebene liegen, der Unterarmteil 32 liegt also in einer Ebene mit dem Oberarmteil 31.

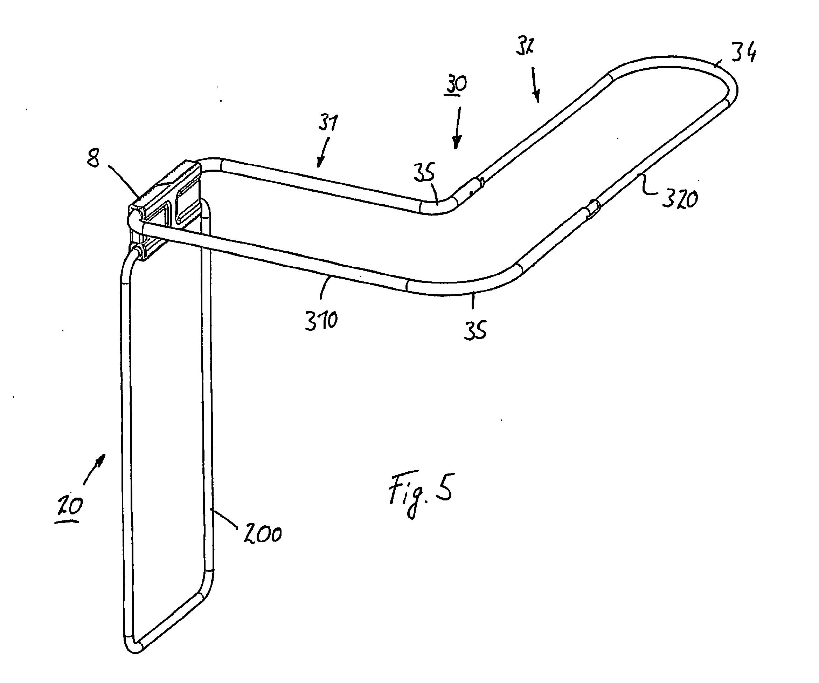

In der Figur 5 ist die Rahmenkonstruktion in einem abgewinkelten Zustand mit einem Abduktionswinkel von 90° dargestellt. Anhand der Figur ist zu erkennen, dass durch einfaches Umlegen des Abstützrahmens 30 um das Gelenk 8 eine Rechts-Links-Vertauschbarkeit realisiert werden kann.

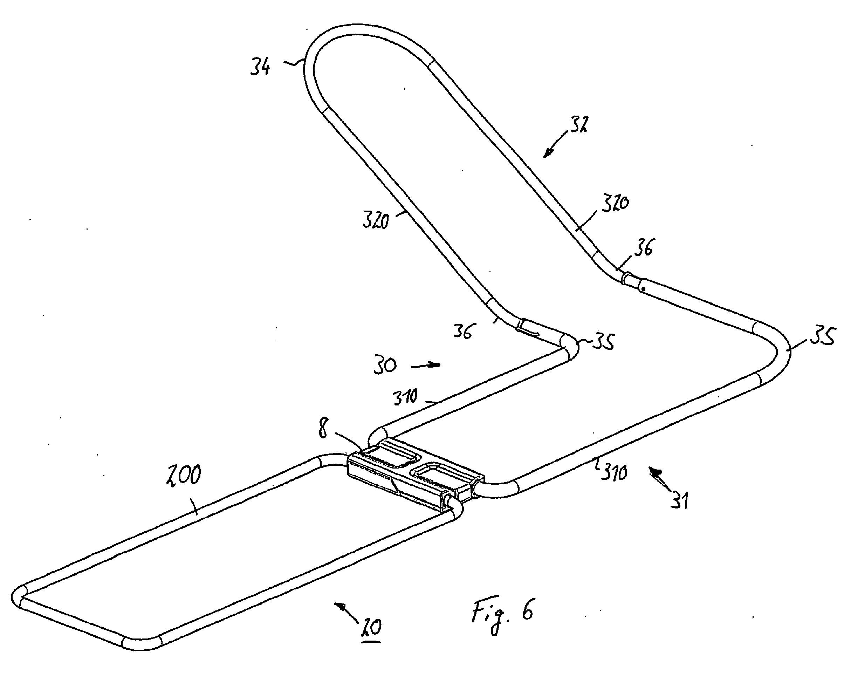

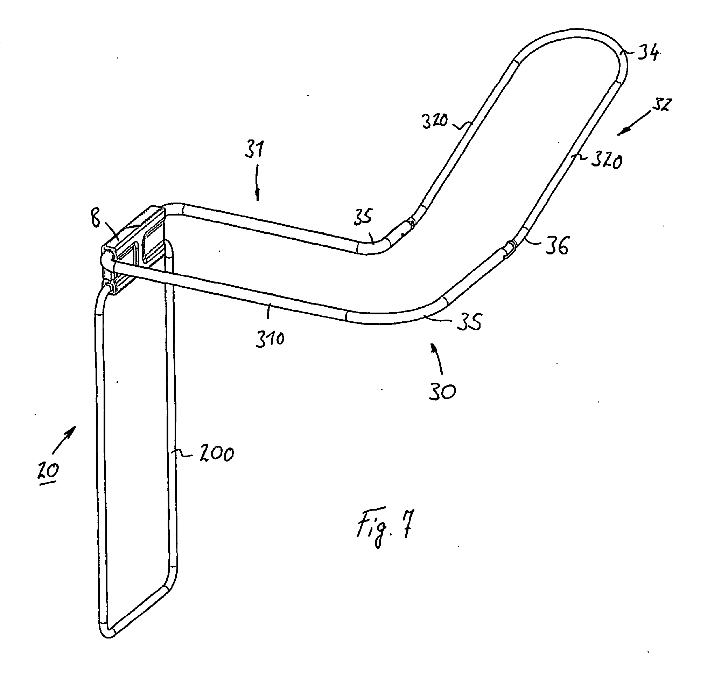

Eine Variante der Erfindung ist in der Figur 6 dargestellt, in der der Unterarmteil 32 in einer Ebene ausgerichtet ist, die schräg zu der Ebene des Oberarmteils 31 liegt. Dies wird durch eine Krümmung 36 in den Endbereichen des Unterarmteils 32 erreicht. Dadurch wird eine Außenrotation des Armes bewirkt, die für einige Versorgungsfälle notwendig ist. Der Außenrotationswinkel von 0° ist in den Figuren 4 und 5 dargestellt, abweichende Außenrotationswinkel sind in den Figuren 6 und 7 gezeigt. Der Außenrotationswinkel kann in einem Bereich zwischen 0° und 30° liegen und wird durch Einsetzen unterschiedlicher Unterarmteile 32 realisiert. Alternativ dazu können gebogene Endstücke in den Oberarmteil 31 eingesetzt werden und als Adapter wirken, so dass nur noch ein gerades Unterarmteil 32 eingesteckt werden muss. Anschließend wird der Bezug 33 oder das Abstützmaterial aufgebracht bzw. übergezogen, um eine angenehme Auflage für den Arm zu schaffen. Auch hier können die Rahmenprofile durch Ineinanderstecken oder durch Steckhülsen aneinander gesichert werden. Um dann eine Rechts-Links-Verwendbarkeit realisieren zu können, müsste ein entsprechender Umbau entweder der Adapter bzw. Winkelstücke oder des Unterarmteils 32 erfolgen, indem der Unterarmteil 32 demontiert, gedreht und entsprechend orientiert wieder an dem Oberarmteil 31 montiert wird.

Abweichend von der dargestellten Ausführungsform mit einem gebogenen Rohrrahmen des Unterarmteils 32 können Außenrotationswinkel auch über Auflagen in Keilform leicht variiert werden.

## Patentansprüche

1. Armabduktionsorthese mit einem zur Abstützung am Thorax des Orthesennutzers vorgesehenen Körperrahmen (20), an dem Befestigungsmittel zur Fixierung des Körperrahmens (20) an dem Orthesennutzer angeordnet sind, und einer Auflageeinrichtung (3) zur Auflage eines Armes des Orthesennutzers, die gelenkig an dem Körperrahmen (20) befestigt ist und Mittel zur Festlegung des Armes an der Auflageeinrichtung (3) aufweist, wobei die Auflageeinrichtung (3) einen Abstützrahmen (30) mit zwei zueinander beabstandeten Rahmenprofilen aufweist, zwischen denen ein Auflagematerial (33) angeordnet ist, und sich der Abstützrahmen (30) an dem Körperrahmen (20) über zumindest ein Stützelement (4) abstützt, **dadurch gekennzeichnet, dass** das Auflagematerial (33) flexibel ausgebildet und zwischen Rahmenprofilen (310, 320) gespannt ist.

2. Armabduktionsorthese nach Anspruch 1, **dadurch gekennzeichnet, dass** der Abstützrahmen (30) geschlossenen ausbildet ist.

3. Armabduktionsorthese nach Anspruch 1 oder 2, **dadurch gekennzeichnet, dass** der Abstützrahmen (30) eine gewinkelte Kontur aufweist.

4. Armabduktionsorthese nach einem der voranstehenden Ansprüche, **dadurch gekennzeichnet, dass** der Körperrahmen (20) eine geschlossene, im Wesentlichen ebene Kontur aufweist.

5. Armabduktionsorthese nach einem der voranstehenden Ansprüche, **dadurch gekennzeichnet, dass** der Abstützrahmen (30) und der Körperrahmen (20) über ein Doppelgelenk (8), zumindest ein Einhakelement oder ein Filmscharnier gelenkig aneinander gelagert sind.

6. Armabduktionsorthese nach einem der voranstehenden Ansprüche, **dadurch gekennzeichnet, dass** der Abstützrahmen (30) an dem Körperrahmen (20) in einem Winkel von 360° verschwenkbar gelagert ist.

7. Armabduktionsorthese nach einem der voranstehenden Ansprüche, **dadurch gekennzeichnet, dass** der Abstützrahmen (30) mehrteilig ausgebildet ist und über Steckverbindungen montiert ist.

8. Armabduktionsorthese nach einem der voranstehenden Ansprüche, **dadurch gekennzeichnet, dass** ein Unterarmteil (32) des Abstützrahmens (30) in einer Ebene liegt, die in einem Winkel zu der durch einen Oberarmteil (31) gebildeten Ebene orientiert ist.

9. Armabduktionsorthese nach einem der voranstehenden Ansprüche, **dadurch gekennzeichnet, dass** der Körperrahmen (20) mit einem flexiblen Bezug (23) bespannt ist.

10. Armabduktionsorthese nach Anspruch 9, **dadurch gekennzeichnet, dass** der Bezug (23) gepolstert ist und insbesondere aus einem kaschierten Schaumstoff besteht.

11. Armabduktionsorthese nach einem der voranstehenden Ansprüche, **dadurch gekennzeichnet, dass** an dem Körperrahmen (20) ein Stabilisierungsabschnitt (9) angeordnet ist.

12. Armabduktionsorthese nach Anspruch 11, **dadurch gekennzeichnet, dass** der Stabilisierungsabschnitt (9) gepolstert ist, insbesondere mit einem kaschierten Schaumstoff.

13. Armabduktionsorthese nach einem der voranstehenden Ansprüche, **dadurch gekennzeichnet, dass** das Abstützmaterial (33) gepolstert ist und insbesondere aus einem kaschierten Schaumstoff besteht.

14. Armabduktionsorthese nach einem der voranstehenden Ansprüche, **dadurch gekennzeichnet, dass** das Stützelement (4) als Keil, Flachelement oder festlegbare Strebe ausgebildet ist.

15. Armabduktionsorthese nach einem der voranstehenden Ansprüche, **dadurch gekennzeichnet, dass** an dem Abstütz- oder Körperrahmen (30, 20) Verstärkungen angeordnet oder ausgebildet sind.

## Claims

1. Arm abduction orthotic with a body frame (20) for support, which rests against the thorax of the orthotic user and which is provided with fastenings for attaching the body frame (20) to the orthotic user, and a support device (3) for supporting an arm of the orthotic user, the support device being joined by a hinge to the body frame (20) and having means for securing the arm to the support device (3), wherein the support device (3) comprises a support frame (30) having two frame profiles at a distance from each other with a support material (33) between them and that the support frame (30) is supported on the body frame (20) by at least one support element (4), **characterized in that** the support material (33) is flexible and stretched between frame profiles (310, 320).

2. Arm abduction orthotic according to claim 1, **characterized in that** the support frame (30) has a closed form.

3. Arm abduction orthotic according to claim 1 or 2, **characterized in that** the support frame (30) has an angled contour.

4. Arm abduction orthotic according to any of the preceding claims, **characterized in that** the body frame (20) has a closed, essentially flat contour.

5. Arm abduction orthotic according to any of the preceding claims, **characterized in that** the support frame (30) and the body frame (20) articulate with one another by means of a double joint (8), at least one hooking element or a film hinge.

6. Arm abduction orthotic according to any of the preceding claims, **characterized in that** the support frame (30) is mounted on the body frame (20) in such a way as to be pivotable through an angle of 360°.

7. Arm abduction orthotic according to any of the preceding claims, **characterized in that** the support frame (30) is multisectional and is assembled by means of pushfit connections.

8. Arm abduction orthotic according to any of the preceding claims, **characterized in that** a forearm part (32) of the support frame (30) lies in a plane that is oriented at an angle to the plane formed by an upper-arm part (31).

9. Arm abduction orthotic according to any of the preceding claims, **characterized in that** the body frame (20) has a flexible cover (23).

10. Arm abduction orthotic according to claim 9, **characterized in that** the cover (23) is padded and, in particular, consists of a laminated foam.

11. Arm abduction orthotic according to any of the preceding claims, **characterized in that** the body frame (20) has a stabilizing section (9).

12. Arm abduction orthotic according to claim 11, **characterized in that** the stabilizing section (9) is padded, in particular with a laminated foam.

13. Arm abduction orthotic according to any of the preceding claims, **characterized in that** the support material (33) is padded and, in particular, consists of a laminated foam.

14. Arm abduction orthotic according to one of the preceding claims, **characterized in that** the support element (4) is formed as a wedge, a flat element, or as a strut that can be fixed in position.

15. Arm abduction orthotic according to one of the preceding claims, **characterized in that** the support frame (30) or body frame (20) is fitted with or formed with strengthening elements.

## Revendications

1. Orthèse d'abduction du bras comprenant, prévu pour l'appui sur le thorax de l'utilisateur de l'orthèse, un châssis corporel (20) sur lequel sont agencés des moyens de fixation pour la fixation du châssis corporel (20) sur l'utilisateur de l'orthèse, et un dispositif de support (3) pour supporter un bras de l'utilisateur de l'orthèse le dispositif de support (3) étant fixé articulé sur le châssis corporel (20) et présentant des moyens pour immobiliser le bras sur le dispositif de support (3), le dispositif de support (3) présentant un châssis d'appui (30) comprenant deux profilés de châssis espacés l'un de l'autre, entre lesquels est agencé un matériau de support (33), et le châssis d'appui (30) s'appuyant sur le châssis corporel (20) par l'intermédiaire d'un moins un élément d'appui (4), **caractérisée en ce que** le matériau de support (33) est formé flexible et est tendu entre les deux profilés de châssis (310, 320).

2. Orthèse d'abduction du bras selon la revendication 1, **caractérisée en ce que** le châssis d'appui (30) est formé fermé.

3. Orthèse d'abduction du bras selon la revendication 1 ou 2, **caractérisée en ce que** le châssis d'appui (30) présente un contour coudé.

4. Orthèse d'abduction du bras selon l'une quelconque des revendications précédentes, **caractérisée en ce que** le châssis corporel (20) présente un contour fermé sensiblement plan.

5. Orthèse d'abduction du bras selon l'une quelconque des revendications précédentes, **caractérisée en ce que** le châssis d'appui (30) et le châssis corporel (20) sont montés articulés l'un par rapport à l'autre par l'intermédiaire d'une double articulation (8), au moins un élément d'accrochage ou un film-charnière.

6. Orthèse d'abduction du bras selon l'une quelconque des revendications précédentes, **caractérisée en ce que** le châssis d'appui (30) est monté au châssis corporel (20), avec possibilité de pivoter sur un angle de 360°.

7. Orthèse d'abduction du bras selon l'une quelconque des revendications précédentes, **caractérisée en ce que** le châssis d'appui (30) est formé en plusieurs pièces et est monté par l'intermédiaire de liaisons enfichées.

8. Orthèse d'abduction du bras selon l'une quelconque des revendications précédentes, **caractérisée en ce qu'**une pièce pour l'avant-bras (32) du châssis d'appui (30) se trouve dans un plan qui est orienté selon un angle par rapport au plan formé par une pièce pour le bras (31).

9. Orthèse d'abduction du bras selon l'une quelconque des revendications précédentes, **caractérisée en ce que** le châssis corporel (20) est garni avec une garniture (23) flexible.

10. Orthèse d'abduction du bras selon la revendication 9, **caractérisée en ce que** la garniture (23) est rembourrée et consiste en particulier en une mousse synthétique stratifiée.

11. Orthèse d'abduction du bras selon l'une quelconque des revendications précédentes, **caractérisée en ce qu'**une partie de stabilisation (9) est agencée sur le châssis corporel (20).

12. Orthèse d'abduction du bras selon la revendication 11, **caractérisée en ce que** la partie de stabilisation (9) est rembourrée, en particulier avec une mousse synthétique stratifiée.

13. Orthèse d'abduction du bras selon l'une quelconque des revendications précédentes, **caractérisée en ce que** le matériau d'appui (33) est rembourré, et consiste en particulier en une mousse synthétique stratifiée.

14. Orthèse d'abduction du bras selon l'une quelconque des revendications précédentes, **caractérisée en ce que** l'élément d'appui (4) est formé par une cale, un élément plat ou des étais pouvant être fixés.

15. Orthèse d'abduction du bras selon l'une quelconque des revendications précédentes, **caractérisée en ce que** des renforts sont formés ou agencés sur le châssis corporel ou d'appui (30, 20).
